# EUROPEAN PATENT SPECIFICATION

(11) **EP 4 308 076 B1**
(45) Date of publication and mention of the grant of the patent: **16.07.2025**
(21) Application number: 22714185.0
(22) Date of filing: 14.03.2022
(51) Int. Cl.: A61K 9/00, A61K 9/08, A61K 9/19, A61K 31/403, A61K 47/18, A61K 49/00

(54) **STABLE FORMULATIONS OF INDOCYANINE GREEN**
STABILE FORMULIERUNGEN VON INDOCYANINGRÜN
FORMULATIONS STABLES DE VERT D'INDOCYANINE

(30) Priority: 17.03.2021 EP 21305324
(43) Date of publication of application: 24.01.2024
(73) Proprietor: Provepharm Life Solutions, 13013 Marseille (FR)
(72) Inventor: LAURENT, Marina, 13013 MARSEILLE (FR); SAYAH, Babak, 13013 MARSEILLE (FR); COURIVAUD, Florian, 13013 MARSEILLE (FR); LOPEZ, Nicolas, 13013 MARSEILLE (FR)
(74) Representative: Bandpay & Greuter
(86) International application number: PCT/EP2022/056446
(87) International publication number: WO 2022/194734

(56) References cited:
- WO-A1-2020/240514
- WO-A1-94/23646
- US-A1- 2018 110 882
- W B?UMLER ET AL: "Photo-oxidative killing of human colonic cancer cells using indocyanine green and infrared light", BRITISH JOURNAL OF CANCER, vol. 80, no. 3-4, 1 January 1999 (1999-01-01), pages 360 - 363, XP055031847, ISSN: 0007-0920, DOI: 10.1038/sj.bjc.6690363

## Description

The present invention relates to the field of medical and pharmaceutical arts. The invention relates to indocyanine green compositions with improved stability, especially improved storage stability. In particular, the invention relates to indocyanine green compositions comprising histidine as defined in the claims.

The present invention also relates to a method for preparing such compositions and methods of use thereof.

### State of the art

Indocyanine green (ICG) is a water-soluble tricarbocyanine fluorescent dye approved by the United States Food and Drug Administration for medical diagnosis. It is used in determining hepatic function, cardiac output, liver blood flow, microcirculation of skin flaps and ophthalmic angiography. These medical uses of ICG are based on its high optical absorbance in a spectral region where human tissues are relatively transparent (750-800 nm). These medical uses are made possible due to its very low toxicity. After intravenous administration, ICG is fast extracted by the liver into bile juice, and has an effective half-life of about 3-4 minutes depending on the hepatic function.

For the above-mentioned applications, ICG has to be administered in the form of aqueous solutions. It is commercially available as a lyophilized powder that can be dissolved in sterile water for injection and is often supplied as a kit. However, ICG has a poor stability in aqueous environments and degrades quickly, which limits its usefulness in certain applications, including time-sensitive surgical procedures. The instructions of vendors of ICG range from immediate use of the reconstituted ICG solution up to usage within ten hours, with any unused portion being discarded.

Studies have shown that in aqueous media, ICG molecules undergo physicochemical transformations such as aggregation, especially at high concentrations. ICG in water readily forms dimers and oligomers while it remains monomeric in human plasma, methanol and dimethyl sulfoxide (DMSO) ^{[1]}. Also, singlet oxygen can be generated by ICG leading to dioxetanes that thermally decompose into several carbonyl compounds ^{[2]}. These decomposition products could reduce the viability of cells in vitro.

The physicochemical transformations of ICG occur more readily in response to light exposure, elevated temperatures, presence of oxygen and at high concentrations of ICG ^{[3, 4, 5]}. The type and intensity of light to which ICG is exposed also affects the degradation. Physicochemical transformations result in the quick degradation of the optical properties of ICG, leading to a discoloration, a diminished fluorescence intensity, and a shift in peak absorbance. It has been reported that the absorbance of ICG at its spectral peak is reduced by 10% after 10 hours of storage in water and direct light ^{[4]}.

In an effort to overcome these shortcomings, many developments have been carried out to improve the stability of Indocyanine green in aqueous solutions.

Engel et al. reported that the chemical degradation of ICG can be inhibited by the addition of sodium azide NaN₃, a quencher of singlet oxygen ^{[2]}.

Mindt et al. investigated the stability of samples of ICG stored either in dark or in clear vials at different temperatures. The absorbance and fluorescence measurements show that, when stored at 4°C in the dark, ICG is stable for three days with 20% fluorescence intensity lost during this period. On the basis of these tests, it is suggested that ICG could be used for longer than one day after preparation from stock if the ICG solution is stored at 4°C in the dark ^{[6]}.

Other researchers have suggested encapsulating ICG into nanometer-sized carriers protecting the ICG molecule from degradation. For example, CN103301482 discloses tri-block polypeptide ICG loaded micelles comprising a polyleucine core, a polylysine middle layer and a polyethylene glycol shell, wherein indocyanine green is dispersed in the core. The core-shell structure to wrap the indocyanine green makes it possible to effectively prevent ICG molecules from being aggregated, so that the stability of ICG is enhanced.

Kirchherr et al. discloses the encapsulation of ICG within Solutol HS15 (polyoxyethylene esters of 12-hydroxystearic acid) micellar system. This formulation exhibited lower aggregation behavior and high aqueous stability over 4 weeks compared to free ICG in aqueous solution ^{[7]}.

US 6,944,493 discloses indocyanine green compositions exhibiting enhanced stability and enhanced indocyanine green concentration. The invention is based on indocyanine green liposomal formulations which are stable for at least 24 hours after reconstitution. All the formulations disclosed in US 6,944,493 are reconstituted with a diluent solution comprising ethanol and polysorbate 80 among others.

WO 2016/123864 discloses compositions comprising ICG embedded in the lipid membrane of a lipid nanoparticle. The nanoparticle has a fluorescence intensity between 4-and 5-fold higher than that of free ICG. After being stored at 4 °C, the nanoparticle has a fluorescence intensity between 4-and 100,000-fold higher than that of free ICG in aqueous solution for between 0 and 300 days.

Dedora et al. discloses the complexation of ICG with highly soluble methyl β-CyD and FDA-approved sulfobutyl ether β-CyD (Captisol ^{®}) in aqueous solution. These complexes allow reducing the aggregation of ICG and exhibit sustained fluorescence increases over 24 hours of more than twice when compared with free ICG in water. However, it has been found that ICG complexed with methyl β-CyD severely reduced the viability of some fibroblasts ^{[8]}.

The encapsulation or the complexation of ICG with the above systems present drawbacks such as incompatibility with the cells, or could limit the ability of the ICG molecules, once injected into the blood, to interact with plasma proteins, and therefore could potentially slow down the excretion kinetics from the vasculature.

US 2010/0181535 discloses a method for enhancing the fluorescence intensity of a fluorescent dye, wherein a fluorescent dye is admixed with a redox buffer. The fluorescent dye can be selected from rhodamines, carborhodamines, oxazine dyes, and/or cyanine dyes including indocyanine green. The redox buffer may comprise carotenoids, in particular tocopherols, thiols, in particular gluthathione, N-acetylcysteine, dihydrolipoic acid, amino acids, in particular tryptophan, tyrosine, histidine, cysteine, methionine and/or peptides and proteins containing them. The authors showed that the use of ascorbic acid or 6-Hydroxy-2,5,7,8-tetramethylchromane-2-carboxylic acid (Trolox^{®}) and methylviologen allows to extend the length of the dark state of a cyanine dye when irradiated with a laser signal in a fluorescence detecting instrument. The authors mention that another advantage of the use of the described redox buffer is that the shelf life of the fluorescent dye solution containing it can be increased by 10% to 20% compared to the otherwise identical fluorescent dye solution without redox buffers. However, this document is silent with regards to the long-term storage stability of the compositions.

WO2020/240514 discloses a formulation of indocyanine green comprising disodium hydrogen phosphate, sodium dihydrogen phosphate and sodium chloride. The compositions may optionally include other pharmaceutically acceptable excipients such as sodium chloride, citrate, sodium carbonate, sodium bicarbonate, tartarate, benzoate, acetate, boric acid, lactic acid, glutaric acid, malic acid, succinic acid and carbonic acid, alkali or alkaline earth salts of any of these acids, buffers of amino acids such as arginine, alanine, histidine, glycine, and lysine. After reconstitution with an aqueous diluent, the solution is stable up to 24 hours.

All of these prior arts report increasing the aqueous stability of ICG, but none provides an aqueous solution of ICG with a stability for a period of time superior to 1-3 days. Thus, a need exists for an ICG aqueous composition that exhibits stability exceeding those of the prior art formulations.

Some of the formulations of the prior art are prepared in expensive equipment, and/or require long manufacturing process times and/or specialized excipients. Some formulations require excipients like alcohol and polysorbate that make the injection of the ICG composition painful and challenging.

In particular, there was a need for an ICG aqueous composition that is stable at room temperature, for periods of time of one or several months.

There was also a need for ICG compositions for intravenous administration that are compatible with target tissues, non-toxic and whose metabolic elimination is rapid.

The invention also aims at providing ICG compositions that are simple to formulate and are based on easily accessible material, and with ICG concentration sufficient to be used in the same manner as prior art short shelf-life ICG formulations.

It has been surprisingly found a formulation of ICG comprising histidine showing improved stability when compared with prior art compositions.

The formulations according to the invention comprise ICG at concentrations comparable or superior to those of the prior art. They can be formulated as a lyophilized powder or as an aqueous solution. They have an improved storage stability and shelf life in comparison with those of the prior art.

It was also an aim of the invention to provide formulations that are stable, cost effective and are easy to manufacture. It was an aim of the invention to provide formulations that do not cause any discomfort to the patient at the time of administration.

### Summary of the invention

The disclosure relates to a composition comprising indocyanine green (a) and histidine (b), wherein the drop in content of indocyanine green in the composition measured in % area by HPLC at 240 nm is less than or equal to 10%, when the composition is stored at ambient temperature for at least 1 month.

Advantageously, the content of ICG in the composition of the disclosure prior to storage is greater than or equal to 90%, the content of indocyanine green being measured as % area by HPLC at 240 nm.

Preferably, the total amount of impurities in the ICG composition prior to storage, measured as area % by HPLC at 240 nm, is less than or equal to 10%.

Preferably, the increase of impurities in the composition according to the disclosure as area % variation measured by HPLC at 240 nm is less than or equal to 10%, when the composition is stored for at least 1 month at ambient temperature.

The invention also relates to a composition comprising indocyanine green (a) and histidine (b), wherein the weight ratio of histidine (b)/indocyanine green (a) is greater than 0.05.

The invention also relates to a kit comprising in separate compartments at least 1/ a composition comprising indocyanine green (a) and histidine (b), wherein the weight ratio of histidine (b)/indocyanine green (a) is greater than 0.05, and 2/ an aqueous diluent.

The invention also relates to a kit comprising in separate compartments at least 1/indocyanine green (a) and 2/ an aqueous solution comprising at least histidine (b), wherein the weight ratio of histidine (b)/indocyanine green (a) in the kit is greater than 0.05.

The invention also relates to a composition comprising indocyanine green (a) and histidine (b), wherein the weight ratio of histidine (b)/indocyanine green (a) is greater than 0.05, for its use as a medicament or a diagnostic agent.

The invention also relates to a method of diagnosis and/or therapy in a patient comprising the administration in an effective amount, in particular by intravenous route, of a composition comprising indocyanine green (a) and histidine (b), wherein the weight ratio of histidine (b)/indocyanine green (a) is greater than 0.05.

Advantageously, the weight ratio histidine (b)/indocyanine green (a) ranges from 0.05 to 50.

According to an embodiment, the weight ratio histidine (b) /indocyanine green (a) ranges from 0.05 to 50, preferably from 0.05 to 40, more preferably from 0.10 to 30, and still more preferably from 0.10 to 20.

Advantageously, the indocyanine green composition according to the invention further comprises at least one compound (c) chosen from ethylenediaminetetraacetic acid (EDTA) or salts thereof, cysteine, sodium chloride, dithiothreitol (DTT) and mixtures thereof.

Preferably, the weight ratio of compound (c) to indocyanine green (a) ranges from 1:500 to 100:1, preferably from 1:100 to 10:1, more preferably from 1:100 to 4:1.

Preferably, the weight ratio of compound (c) to histidine (b) ranges from 1:100 to 100:1, preferably from 1:10 to 10:1.

According to an embodiment, the composition comprises up to 5% by weight of sodium iodide based on the weight of indocyanine green.

According to a preferred embodiment, the indocyanine green composition according to the invention is an aqueous composition.

According to an even preferred embodiment, the indocyanine green composition according to the invention is an aqueous composition packed in a vial or an ampoule.

Advantageously, the concentration of indocyanine green in the aqueous composition is comprised between 0.1 and 50 mg/ml, preferably between 0.1 and 30 mg/ml.

According to an embodiment, the aqueous composition is reconstituted from a kit comprising in separate compartments at least 1/ a composition comprising indocyanine green (a) and histidine (b), wherein the weight ratio of histidine (b)/indocyanine green (a) is greater than 0.05, and 2/ an aqueous diluent.

According to another embodiment, the aqueous composition is reconstituted from a kit comprising in separate compartments at least 1/indocyanine green (a) and 2/ an aqueous solution comprising histidine (b), wherein the weight ratio of histidine (b)/indocyanine green (a) is greater than 0.05.

Advantageously, the concentration of indocyanine green in the aqueous composition reconstituted from any of the kits described above and in details here-under is comprised between 0.1 and 50 mg/ml, preferably between 0.1 and 30 mg/ml.

According to another embodiment, the composition is in the form of a lyophilized powder.

### Detailed description

The term "consists essentially of" followed by one or more characteristics, means that may be included in the process or the material of the invention, besides explicitly listed components or steps, components or steps that do not materially affect the properties and characteristics of the invention.

The expression "comprised between X and Y" includes boundaries, unless explicitly stated otherwise. This expression means that the target range includes the X and Y values, and all values from X to Y.

The references to methods of treatment in the description can be interpreted as references to the compounds, pharmaceutical compositions and medicaments of the present invention for use in a method for the treatment of the human (or animal) body by therapy or for diagnosis.

### The stability of indocyanine green composition according to the invention

The present invention is intended to provide a composition of indocyanine green with improved stability compared to prior art compositions.

The terms "stable composition" and "stability" refer to a composition wherein the indocyanine green content remains stable upon storage during a certain amount of time (for example 1 week, 1 month, 2 months, 6 months, 1 year, 2 years, etc...).

The stability of the composition is evaluated by measuring the variation of indocyanine green content in the composition after storage during extended periods of time.

Preferably, the compositions according to the invention are advantageous in that they show substantially no signs of aggregation, degradation, precipitation and substantially no, or minimal, formation of impurities as a result of chemical modification of the indocyanine green after storage during extended periods of time. In addition, the compositions according to the invention are advantageous in that they show no significant discoloration or decrease in fluorescence intensity or in peak absorbance after storage during extended periods of time.

In particular, the present invention provides a composition comprising indocyanine green that exhibits improved stability as compared to presently available indocyanine green formulations.

Advantageously, the composition according to the invention exhibits a stability of at least 10, and more preferably at least 30, more preferably at least 50, more preferably still 100 times relative to the currently-approved indocyanine green formulations.

In one aspect, the composition according to the invention is stable when stored at ambient temperature for at least one month, preferably for at least 3 months, more preferably for at least 6 months, still more preferably for at least 12 months and most preferably for at least 2 years.

The term "ambient temperature" means a controlled room temperature as between 20 to 25 °C, with excursions between 15 to 30 °C, as defined by the United States Pharmacopeia, or between 15 to 25 °C as defined by the European Pharmacopoeia.

Preferably, the composition according to the invention is stable at ambient temperature over a period of time ranging from 1 month to 3 years, preferably over a period ranging from 3 months to 3 years, more preferably over a period ranging from 1 year to 3 years, still more preferably over a period ranging from 1 year to 2 years at ambient temperature.

Advantageously, the composition according to the invention is stable when stored in a container protecting it from natural light and/or UV light and/or fluorescent light for at least one month, preferably for at least 3 months, more preferably for at least 6 months, and most preferably for at least 2 years.

A container capable of protecting the composition from natural light and/or UV light and/or fluorescent light is a container the walls of which have a maximum percentage of light transmission at any wavelength of at least 50%. Typically, the container can be an opaque or amber-colored container and/or an opaque secondary packaging component (e.g., carton or overwrap).

Advantageously, the composition according to the invention is stable when stored in a tightly closed or hermetically closed container for at least one month, preferably for at least 3 months, more preferably for at least 6 months, and most preferably for at least 2 years.

The stability of the composition according to the invention can be evaluated by visual inspection of color and/or transparency and/or other analytical methods.

The analytical methods for evaluating the stability of the composition are known by the person skilled in the art. They can include nuclear magnetic resonance (NMR), high-performance liquid chromatography (HPLC), size exclusion chromatography (SEC), liquid chromatography coupled with mass spectrometric analysis (LC-MS), dynamic light scattering (DLS), differential scanning calorimetry (DSC), UV spectroscopy, and Fourier-transform infrared spectroscopy (FTIR) or a combination of these methods.

Advantageously, the stability of the composition is evaluated by measuring the variation of indocyanine green content in the composition by HPLC before and after storage during extended periods of time. More advantageously, the ICG content in the composition and the variation of this content is evaluated in % area by HPLC at 240 nm.

Preferably, the stability of the inventive composition corresponds to a drop in content of indocyanine green in the composition measured in % area by HPLC at 240 nm of less than or equal to 10 %, when the composition is stored at ambient temperature for at least 1 month, preferably 3 months, more preferably 6 months, still more preferably 12 months and most preferably for at least 2 years.

Preferably, evidence of the stability of the inventive composition corresponds to a drop in content of indocyanine green in the composition measured in % area by HPLC at 240 nm of less than or equal to 5%, when the composition is stored at ambient temperature for at least 1 month, preferably 3 months, more preferably 6 months, still more preferably 12 months and most preferably for at least 2 years.

Even more preferably, evidence of the stability of the inventive composition corresponds to a drop in content of indocyanine green in the composition measured in % area by HPLC at 240 nm of less than or equal to 2%, when the composition is stored at ambient temperature for at least 1 month, preferably 3 months, more preferably 6 months, still more preferably 12 months and most preferably for at least 2 years.

By "drop in content" it is to be understood, within the meaning of the present application, the percentage difference between the measured content of ICG in the composition prior to storage and the measured content of ICG in the composition after a determined storage time, the content being measured in % area by HPLC at 240 nm.

Preferably, the measured content of ICG in the composition prior to storage is greater than or equal to 90%, preferably greater than or equal to 95%, more preferably greater than or equal to 97%, most preferably greater or equal to 99%, the content of indocyanine green being measured as % area by HPLC at 240 nm.

Stability of the indocyanine green inventive composition may also correspond to the fact that the total amount of impurities contained in the inventive composition does not increase during storage, in particular does not increase to a level that reduces the capability of the indocyanine green composition to be used for medical and/or diagnostic applications.

For example, when the composition is stored at ambient temperature for at least 1 month and such impurities are then measured by HPLC, only a small amount of the impurities in the composition is observed and this amount does not increase considerably over time.

The term "impurity" is to be understood, within the context of the present invention, as a chemical entity that is not indocyanine green, or excipients or other additives to the indocyanine green inventive composition.

The impurities may be process-related impurities such as byproducts or intermediates that can be formed during the manufacture of the indocyanine green, or degradation related impurities that result from chemical transformation of indocyanine green during storage. Degradation related impurities are products that may be formed during storage, for example in response to light exposure, temperature, and humidity, or reaction with oxygen.

In an embodiment, the increase of impurities in the composition according to the invention as area % variation measured by HPLC at 240 nm is less than or equal to 10%, preferably less than or equal to 5%, more preferably less than or equal to 2% when the composition is stored for at least 1 month, preferably 2 months, more preferably 6 months, most preferably for at least 2 years at ambient temperature.

In an embodiment, the total amount of impurities in the ICG composition prior to storage, measured as area % by HPLC at 240 nm, is less than or equal to 10%, preferably less than or equal to 5%, more preferably less than or equal to 1%.

The term "total amount of impurities" includes process related impurities and degradation related impurities as described above.

### The composition of indocyanine green

The disclosure provides a composition comprising indocyanine green (a) and histidine (b).

Surprisingly, the inventors have observed that histidine is capable of stabilizing, and/or avoiding degradation of, and/or reducing degradation of, and/or preventing degradation of, and/or improving shelf life, and/or increasing shelf life of a composition of ICG in an aqueous diluent.

Surprisingly, the inventors have observed that the association of histidine with some other compounds is capable of improving the stabilizing effect of histidine towards ICG in an aqueous diluent.

Advantageously, the composition according to the invention may comprise at least one co-stabilizing compound (c).

### The indocyanine green (a)

The term indocyanine green refers to indocyanine green as well as pharmaceutically acceptable salts, solvates, hydrates, acids, anhydrous and free base forms thereof, preferably it refers to indocyanine green.

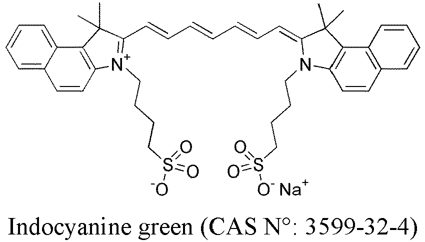

Indocyanine green can be prepared by any process known to those skilled in the art. For example, indocyanine green can be prepared according to the process described in WO95/07888 or WO2017093889. Preferably, the indocyanine green preparation process involves a step of purification of the obtained product.

Advantageously, the indocyanine green used in the composition according to the invention is substantially pure. Preferably, the indocyanine green used in the composition according to the invention is provided as a composition with a purity greater than 90.0%, preferably greater than 95.0 %, more preferably greater than 97.0%, still more preferably, greater than 99.7% as measured by HPLC.

The term "purity" used herein means the extent to which the raw material indocyanine green is free from undesirable or adulterating chemical entities.

According to the invention, purity and impurities in the indocyanine green material are evaluated in % area by HPLC at 240 nm.

Preferably, the indocyanine green used in the composition according to the invention is provided as a composition comprising less than 5.0%, preferably less than 1.0%, more preferably less than 0.5% of impurities as measured by HPLC.

Preferably, the indocyanine green used in the composition according to the invention is provided as a composition comprising less than 0.50%, preferably less than 0.40%, more preferably less than 0.20%, most preferably less than 0.15% of each impurity as measured by HPLC.

The impurities contained in the indocyanine green can be any reaction by-product or intermediate resulting from the process of manufacture. For example, as described in the document US2019/0337896, the impurities can include N-phenylacetamide, 4-(1,1,2-trimethyl-1H-benzo[e]indolium-3-yl) butane-1-sulfonate, 4-(1,1,2-dimethyl-2-((1E, 3E, 5E)-6-(N-phenylacetamido) hexa-1,3,5-trienyl)-1H-benzo[e]indolium-3-yl) butane-1-sulfonate, but are not limited thereto.

Preferably, the indocyanine green is provided as a composition comprising a content of metallic contaminants of less than 200 ppm, preferably, less than 100 ppm, more preferably less than 50 ppm, even more preferably less than 20 ppm, by weight of metals based on weight of indocyanine green. The metallic contaminant content in the indocyanine green can be measured by Inductively Coupled Plasma Mass Spectrometry (ICP-MS).

By "metallic contaminants" it is to be understood the so-called "heavy" metals and in particular: Al, As, Cd, Cr, Cu, Fe, Sn, Mn, Hg, Mo, Ni, Pb, Zn as well as their organic and inorganic derivatives.

Still more preferably, the indocyanine green comprises no or less than 2 ppm of lead and Arsenic.

The indocyanine green used in the composition of the invention is provided as a composition that can also comprise sodium iodide. The sodium iodide is an additive often present in indocyanine green commercial compositions. When present, the iodide content is desirably limited to less than 5% by weight based on the weight of indocyanine green. The sodium iodide content in the indocyanine green can be measured by potentiometry.

The indocyanine green used in the composition of the invention may be provided in any suitable form such as a lyophilizate or a crystalline powder.

### The histidine (b)

By "histidine" it is meant, for the purpose of this invention, the L or the D-enantiomer of the amino acid histidine. Advantageously, histidine means the L-enantiomer of the amino acid histidine.

The inventors have surprisingly observed an enhanced stability of the indocyanine green composition in an aqueous medium, when histidine (b) is associated to indocyanine green (a).

In a preferred embodiment, the weight ratio of histidine (b) to indocyanine green (a) is greater than 0.05. Advantageously, the weight ratio of histidine (b) to indocyanine green (a) ranges from 0.05 to 50, preferably from 0.10 to 40, more preferably from 0.10 to 30, and still more preferably from 0.10 to 20. When the weight ratio of histidine (b) to indocyanine green (a) is too high, it has been observed that histidine precipitates during storage.

In a favorite embodiment of the present invention, the composition consists essentially of indocyanine green (a) and histidine (b), wherein the weight ratio of histidine (b)/indocyanine green (a) is greater than 0.05.

In another favorite embodiment of the present invention, the composition consists essentially of indocyanine green (a), histidine (b), and iodide, wherein the weight ratio of histidine (b)/indocyanine green (a) is greater than 0.05.

According to another aspect, the present invention relates to the use of histidine to improve the storage stability of an ICG composition wherein the weight ratio of histidine (b) to indocyanine green (a) in the composition is greater than 0.05, preferably between 0.05 and 50, preferably between 0.10 and 40, more preferably between 0.10 and 30, and still more preferably between 0.10 and 20.

### The co-stabilizing compound (c)

In another embodiment of the present invention, the composition may further comprise one or more compounds (c).

Advantageously, the compound (c) may be selected from ethylenediaminetetraacetic acid (EDTA) and salts thereof, cysteine, sodium chloride, dithiothreitol (DTT) and mixtures thereof.

By " mixtures thereof" is meant the mixtures of two or more of the above cited compounds in all proportions.

Salts of ethylenediaminetetraacetic acid include, non limitatively, disodium EDTA, calcium sodium EDTA, tetrasodium EDTA, dicalcium EDTA and mixtures thereof. In a preferred embodiment, the salt of EDTA is selected from tetrasodium EDTA and calcium sodium EDTA or mixtures thereof. These salts of ethylenediaminetetraacetic acid may also be in the form of hydrated salts.

By "cysteine" it is meant, for the purpose of this invention, the L or the D-enantiomer of the amino acid cysteine. Advantageously, cysteine means the L-enantiomer of the amino acid cysteine.

The inventors have surprisingly observed an enhanced stability of the indocyanine green composition in an aqueous medium, when the compound (b) and the compound (c) are associated to indocyanine green (a).

Preferably, the weight ratio of compound (c) to indocyanine green (a) ranges from 1:500 to 100:1, preferably from 1:500 to 10:1, more preferably from 1:100 to 10:1, most preferably from 1:100 to 4:1.

Preferably, the weight ratio of compound (c) to compound (b) ranges from 1:100 to 100:1, preferably from 1:10 to 10:1.

The co-stabilizing compound may be provided in any form such as solid, liquid, or semi-solid. It may be also in the form of an aqueous solution.

In a favorite embodiment of the present invention, the composition consists essentially of indocyanine green (a), histidine (b), and a co-stabilizing compound (c) selected from ethylenediaminetetraacetic acid (EDTA) and salts thereof, cysteine, sodium chloride, dithiothreitol (DTT) and mixtures thereof.

In another favorite embodiment of the present invention, the composition consists essentially of indocyanine green (a), histidine (b), iodide and a co-stabilizing compound (c) selected from ethylenediaminetetraacetic acid (EDTA) and salts thereof, cysteine, sodium chloride, dithiothreitol (DTT) and mixtures thereof.

### Other additives

The composition according to the invention may further comprise at least one pharmaceutically acceptable additive.

As used herein, the term "pharmaceutically acceptable" refers to compounds, materials and compositions which are suitable for use in contact with the tissues of human beings and animals without excessive toxicity, irritation, or other problem or complication, commensurate with a reasonable benefit/risk ratio.

The term "additives" include components contained in the composition other than the ingredients (a), (b) and (c) as defined above or sodium iodide. Examples of such additives include buffer agents, pH adjusting agents, isotonizing agents, surfactants, antiseptic agents, tonicifying agents, antimicrobial agents, wetting agents, and emulsifiers.

For example, the composition of the invention may include one or more additive selected from the group comprising sodium hydroxide, potassium hydroxide, methylparaben, propylparaben, sodium acetate, sodium citrate, sodium carbonate, ammonium carbonate, sodium bicarbonate, benzoate, acetate, boric acid, lactic acid, glutaric acid, malic acid, succinic acid and carbonic acid, as well as alkali or alkaline earth salts of these acids, meglumine, hydrochloric acid, cyclodextrine derivatives, sorbitan monolaurate, triethanolamine acetate, triethanolamine oleate, citric acid, lactic acid, phosphoric acid, sulfuric acid, arginine, alanine, glycine and lysine.

These additives are generally available to one of ordinary skill in the art and may be in any form such as solid, liquid, or semi-solid.

Preferably, the amount of additives in the composition of the invention is within a range that does not substantially adversely affect the activity of the indocyanine green. Preferably, the composition of the invention contains the minimum number and amount of additives necessary to provide a stable and efficacious composition.

### Form of the composition according to the invention

The composition according to the invention may be provided in any suitable form, but is preferably provided in the form of an aqueous composition or a lyophilized solid composition.

### Aqueous composition

According to a first aspect, the composition according to the invention is in the form of an aqueous composition.

By "aqueous composition" it is to be understood an aqueous solution, an aqueous suspension, a colloidal aqueous suspension, an aqueous dispersion, preferably an aqueous solution.

The term "aqueous solution" means a composition of indocyanine green as described above, the ingredients of which are soluble in an aqueous medium.

Advantageously, according to this variant, the composition comprises an aqueous diluent, indocyanine green (a), histidine (b), optionally one or more compounds (c) as defined above and optionally one or more pharmaceutically acceptable additive.

The aqueous diluent is a pharmaceutically acceptable solvent, that is safe and nontoxic upon administration to humans or animals, and is useful for the preparation of a pharmaceutical formulation.

The aqueous diluent includes water and may include one or more pharmaceutically acceptable additive. In some variants of the method disclosed here-under, histidine (b) as defined above, optionally one or more compounds (c) as defined above are introduced in the aqueous diluent before mixing with the ICG. In such embodiments, the histidine (b), optionally one or more compounds (c) may be considered as part of the aqueous diluent.

An appropriate aqueous diluent can be any liquid which is biologically acceptable and in which the composition of the invention is completely soluble. Water, particularly sterile water for injection (SWFI) and/or bacteriostatic water for injection (BWFI), is a preferred diluent, since it does not include salts or other compounds which may affect the stability of the ICG. However, another aqueous diluent may be used such as a sterile saline solution, Ringer's solution, dextrose solution, glucose solution and the like.

According to an embodiment, the aqueous diluent is chosen from the group consisting of water for injection, bacteriostatic water for injection, sterile saline solution, Ringer's solution, dextrose solution, and glucose solution.

The person skilled in the art is able to choose the diluent according to the desired use and with respect to the other compounds of the composition. He is also able to adjust the amounts of the excipients according to their solubility in the aqueous solution.

In a preferred embodiment, the aqueous diluent is water for injection, particularly sterile water for injection (SWFI) and/or bacteriostatic water for injection (BWFI).

The aqueous diluent may also comprise buffers, bacteriostats and solutes which render the composition isotonic with the blood of the intended recipient.

According to an embodiment, the aqueous diluent is not a buffer solution or does not contain buffer agents.

Advantageously, the aqueous solution has an osmolarity of at least 1 mOsM. More advantageously, the aqueous solution has an osmolarity comprised between 1 and 300 mOsM, more preferably between 1 and 200 mOsM.

The concentration of indocyanine green in the aqueous composition of the invention can be any concentration that is appropriate for use in medical treatment or diagnostics, in particular to produce angiographic images of satisfactory quality.

According to a favorite embodiment, the aqueous composition according to the invention has a concentration of indocyanine green comprised between 0.1 mg/ml and 50 mg/ml, preferably between 0.1 and 30 mg/ml.

When the ICG solution is of a concentration of less than 0.1 mg/ml, fluorescence is not sufficient for satisfactory detection by analytical instruments.

According to a favorite embodiment, the aqueous composition according to the invention has a concentration of indocyanine green comprised between 0.1 and 25 mg/ml, preferably between 1 and 25 mg/ml, more preferably between 1 and 10 mg/ml.

In the case where one or more constituents of the composition of the invention such as histidine (b) or possibly compound (c) are added as an aqueous solution, the volume of aqueous diluent can be adjusted to obtain the desired concentration of ICG.

In an embodiment of the present invention, the aqueous composition consists essentially of an aqueous diluent, indocyanine green (a), and histidine (b).

In another embodiment, the aqueous composition consists essentially of an aqueous diluent, indocyanine green (a), histidine (b) and sodium iodide.

In another embodiment, the aqueous composition consists essentially of an aqueous diluent, indocyanine green (a), histidine (b) and a co-stabilizing compound (c) selected from ethylenediaminetetraacetic acid (EDTA) and salts thereof, cysteine, sodium chloride, dithiothreitol (DTT) and mixtures thereof.

In another embodiment, the aqueous composition consists essentially of an aqueous diluent, indocyanine green (a), histidine (b) and a co-stabilizing compound (c) selected from ethylenediaminetetraacetic acid (EDTA) and salts thereof, cysteine, sodium chloride, dithiothreitol (DTT) and mixtures thereof, and sodium iodide.

In these embodiments, the favorite percentages of compounds (ICG (a), histidine (b) and co-stabilizing compounds c)) in the aqueous composition are the same as expressed above in the chapter "the composition of indocyanine green".

### Lyophilized solid composition

According to another favorite embodiment, the composition according to the invention is in the form of a lyophilized composition or a lyophilized powder.

The term "lyophilized composition ", "lyophilized powder " or "lyophilizate" refers to the solid composition according to the invention obtained by lyophilization of an aqueous solution.

The term "lyophilization", or "lyophilized," refers to a process by which the composition in an aqueous solvent is first frozen and then the frozen solvent is removed by sublimation in a vacuum environment.

When provided as a lyophilizate, the composition according to the invention can be transformed into a solution prior to administration by use of an aqueous diluent as described therein. The reconstituted aqueous solution, also a composition according to the invention, is then stable at ambient temperature for at least 1 month, preferably 2 months, more preferably 6 months, most preferably for at least 2 years.

All limitations described above regarding the aqueous diluent, ICG concentrations, pH, osmolarity and stability also apply for the aqueous solution reconstituted from the lyophilized composition according to the invention.

Further, in an embodiment, the lyophilized composition according to the invention has a short reconstitution time of less than 2 minutes, preferably less than 1 minute, and is suitable for intravenous administration after dilution with an aqueous diluent.

By "reconstitution time" it is meant the time required to rehydrate the lyophilized composition with an aqueous solution to a particle-free clarified solution.

After the composition has been reconstituted as an aqueous solution for medical or diagnosis use, preferably, the solution is stored in a container that protects it from light and oxygen.

### Kits

According to a favorite embodiment, the invention relates to a kit comprising in a first part the composition according to the invention in the form of a lyophilized powder, and in a second part an aqueous diluent.

According to another favorite embodiment, the invention relates to a kit comprising in a first part indocyanine green (a), and in a second part an aqueous solution comprising histidine (b). Preferably, the aqueous solution comprises a co-stabilizing compound (c) as defined above, and optionally one or more additives as described above.

According to this embodiment, the indocyanine green is preferably in the form of lyophilizate powder.

All the preferred embodiments described above for the ICG composition and the aqueous diluent apply also to any of the kit variants.

According to an embodiment, the kit according to the invention is composed of parts (power and diluent) in amounts such that the aqueous solution reconstituted from the kit has an ICG concentration ranging from 0.1 to 50 mg/ml, preferably from 0.1 to 30 mg/ml, more preferably from 0.1 and 25 mg/ml, still more preferably comprised between 1 and 25 mg/ml, and most preferably between 1 and 10 mg/ml.

According to these favorite embodiments, the kit comprises at least two separate compartments where the lyophilized powder and the aqueous diluent are stored separately.

The kit can comprise at least two separate vials, packaged with or without a syringe, or it can comprise a pre-filed two-compartment syringe, like for example a dualchamber by-pass syringe.

Such a kit permits mixing the powder(s) and the aqueous solution before administration.

The kits according to one or the other variant, are intended to be used to reconstitute extemporaneously a stable aqueous solution of indocyanine green as described therein. They have the advantage of providing the essential components of the composition for injection in optimized storage conditions and in appropriate amounts to provide the desired concentration. Thus, the aqueous composition can be reconstituted without dilution by a diluent in addition to the diluent contained in the kit. Thanks to this packaging as a kit, dosing errors, or the introduction of contaminants, may be avoided.

### Method of preparation of the composition according to the invention

The composition according to the invention may be prepared using any known method, and is not limited to specific methods.

For example, the composition may be prepared by mixing the indocyanine green (a) with histidine (b), and optionally with one or more compounds (c) as described above, and optionally one or more pharmaceutically acceptable excipients.

Preferably, the mixing of the ingredients of the composition according to the invention is carried out in an aqueous solvent as described above. The order of addition of the components constituting the composition according to the invention may vary. For example, indocyanine green may be solubilized in the aqueous diluent and histidine b) and optionally the co-stabilizing compound (c) and additives can be added afterwards. The compounds (b) and (c) can be mixed prior to their addition to the solubilized indocyanine green. Alternately, indocyanine green can be introduced into an aqueous solution wherein histidine b) and optionally compound(s) (c) have been previously solubilized.

Preferably, mixing is carried out under inert atmosphere such as under an argon or a nitrogen atmosphere. The aqueous diluent and/or liquid ingredients are preferably degassed before use.

When provided as a lyophilizate, the lyophilization of the composition according to the invention can be carried out using standard equipment for lyophilization or vacuum drying. The cycle of lyophilization may be varied depending on the equipment and facilities used which can be adjusted by those skilled in the art.

The pre-lyophilization aqueous solution is preferably the aqueous solution of indocyanine green as described above. This pre-lyophilization aqueous solution according to the invention can be sterilized prior to lyophilization. The sterilization is generally achieved by filtration of the solution through an appropriate membrane.

Preferably, the aqueous solution is lyophilized in a short delay after its preparation in order to prevent any degradation of the composition. The lyophilizate can be stored for months before use. Preferably the lyophilizate is stored in conditions wherein it is protected from air and/or humidity and/or light.

When provided as an aqueous composition, the indocyanine green composition according to the invention can be also reconstituted by use of the lyophilizate and the aqueous diluent from the kits described above. To carry out the reconstitution, the lyophilizate composition may be introduced into a vial, with the aqueous diluent being added thereafter.

The composition prepared in a lyophilized or aqueous form is advantageously placed in an airtight sealed container protected from light immediately after its preparation. The container may be for example a bottle, an ampoule, a vial, a syringe, or a tube. The container may be formed for example of glass, a polymer, a metal, or the like. The container may be a single-dose or multi-dose container.

In a favorite embodiment of the present invention, the container is preferably tinted glass vials or tinted glass ampoules. Advantageously, it is selected from tinted glass vials having vacuum headspaces or tinted glass ampoules. Preferably, the composition according to the invention is stored in tinted glass ampoules.

Advantageously, the composition according to the invention is stored at ambient temperature.

### Uses of the compositions according to the invention

The compositions of the present invention may be used in a method of diagnosis and/or therapy (as a treatment agent) in a patient. The method comprises the administration of an aqueous solution according to the invention in an effective amount. This administration may be carried out by enteral route, parenteral route, in particular intravenous injection or by local application. Preferably, administration is carried out by intravenous injection.

The term "effective amount" means that amount of indocyanine green that will elicit the biological or medical response of a tissue, system, animal or human that is being sought, for instance, by a researcher or clinician.

Advantageously, the compositions of the present invention can be used in a method of diagnosis and/or therapy wherein the aqueous ICG composition is administered to the patient after being stored for at least 1 month, preferably for at least 6 months, more preferably for at least 12 months and most preferably for at least 2 years from its preparation or reconstitution from one of the kits as described above.

In particular, the composition according to the invention can be used in hyperthermal therapy, selective photocoagulation reinforced by ICG, photodynamic therapy (PDT), photodynamic and hyperthermal therapy (PHT). More particularly, as PDT applications, the composition according to the invention can be used for infection treatment, acne treatment, macular surgery, cancer treatment, etc. The use of the composition according to the invention may be associated with other therapies such as immunotherapy, radiotherapy, ultrasound and chemotherapy.

The composition according to the invention can also be used for obtaining an angiographic image of tissue in a patient, for determining cardiac output, for determining hepatic function and liver blood flow, for the diagnosis and treatment of age-related macular degeneration, of related choroidal neovascularization and tumors.

The compositions according to the invention are based on components which have been tested clinically and approved for administration to humans and/or animals.

For diagnosis applications, the amount of ICG composition administered to a patient should be sufficient to permit the dye to fluoresce when irradiated at the appropriate wavelength, knowing that the peak absorption and emission of ICG lies in the range of 800-850 nm. The same standard is applicable to the therapeutic methods using ICG solution; sufficient dye should be administered to enable the treatment to be efficient. The amounts of ICG composition for administration may be readily determined by those skilled in the art, and should be at least the concentration currently accepted for use in ophthalmic angiography, e.g., for diagnosis, 2 ml of a 20 mg/mL ICG solution. As acknowledged by the skilled professional, higher dye concentrations may advantageously be used in any of these diagnostic and treatment methods.

The improved stability of ICG in the solutions according to the invention provides improved diagnosis/treatment of the patient with the same amount of injected compounds, in comparison with prior art ICG compositions. This is made possible because the improved stability of ICG will provide a more intense response to fluorescence irradiation.

### Examples

### I- Materials and methods:

### 1. Reagents and chemicals:

Indocyanine green was supplied by Biophore India Pharmaceuticals and used without further purification. Cysteine (CAS number: 3374-22-9) was provided by Fisher Scientific. Sodium ascorbate (CAS number: 134-03-2) and histidine (CAS number: 4998-57-6) were provided by Aldrich. The other reagents were purchased from Aldrich and used without further purification.

### 2. Equipment:

The stability of formulations in the foregoing examples was determined by high pressure liquid chromatography (HPLC). The equipment used to perform the analysis was an Agilent Infinity 1260 HPLC/MS system equipped with a diode array detector (UV-visible), Waters Xbridge Shield RP18- 4.6x100-5µm column and a MS ESI spectrometer. The injected volume of solution was 5 µL. Flow rate was 1 ml/min, column temperature was 30 °C, and detection was at 240 nm.

The elution is carried out by gradient. The mobile phase consists of a mixture of ammonium acetate/acetic acid buffer 10 mM pH 5.5 (eluent A) and acetonitrile (eluent B). The composition of the mobile phase was modified continuously during the elution process according to the following Table I:

**Table I**

| **Time (min)** | **Eluent A%** | **Eluent B%** |
|---|---|---|
| 0.0 | 85 | 15 |
| 3.0 | 85 | 15 |
| 40.0 | 30 | 70 |
| 40.1 | 85 | 15 |
| 47.0 | 85 | 15 |

### II- Sample preparation:

The ICG compositions below were prepared as follows: histidine and, when present, the co-stabilizing compound are weighed into a type I clear glass vial. The vial is then tared and 25 mg of indocyanine green is weighed. The vial is closed with a bromobutyl rubber stopper (from VWS) and then purged with a flow of nitrogen. 10 ml of WFI previously degassed with nitrogen are then added with a syringe. The vials are gently agitated manually until the solubility of the solutes is reached. The vials are then stored at room temperature and away from light.

### III- Compositions comprising indocyanine green (a) and histidine (b):

**Table II**

| | C1 | C2 | C3 |
|---|---|---|---|
| ICG (mg) | 25 | 25 | 25 |
| Histidine (mg) | 16 | 200 | 300 |
| Weight ratio histidine/ICG | 0.64 | 8 | 12 |

| Storage stability | | | |
|---|---|---|---|
| % area ICG prior to storage (i) | 98.26 | 99.70 | 99.70 |
| Storage (weeks) | 6 | 5 | 5 |
| % area ICG after storage (f) | 97.45 | 98.06 | 97.86 |
| Variation (i-f) % | 2.25 | 1.64 | 1.84 |

The results reported in Table II show that the stability of the aqueous indocyanine green composition is enhanced in presence of histidine.

### IV- Compositions comprising indocyanine green (a), histidine (b) and a co-stabilizing compound (c):

**Table III**

| | | C4 | C5 | C6 | C7 |
|---|---|---|---|---|---|
| ICG (mg) | | 25 | 25 | 25 | 25 |
| Histidine (mg) | | 5 | 5 | 5 | 5 |
| Compound (c) | Cysteine (mg) | 4 | 4 | | |
| | NaCl (mg)^{(a)} | | 45 | 45 | |
| | EDTA 4Na (mg) | | | 1 | 1 |
| | DTT (mg) | | | | 4 |
| Weight ratio histidine/ICG | | 0.20 | 0.20 | 0.20 | 0.20 |
| Weight ratio compound (c)/ICG | | 0.16 | 1.96 | 1.84 | 0.20 |

| Storage stability | | | | | |
|---|---|---|---|---|---|
| % area ICG prior to storage (i) | | 99.07 | 99.07 | 98.26 | 99.07 |
| Storage (weeks) | | 5 | 1 | 7 | 5 |
| % area ICG after storage (f) | | 98.43 | 98.21 | 97.29 | 98.35 |
| Variation (i-f) % | | 0.64 | 0.86 | 0.97 | 0.72 |

| | | | | | |
|---|---|---|---|---|---|
| ^{(a)} To prepare solutions comprising sodium chloride, the quantities of ICG, histidine and cysteine/ EDTA /sodium ascorbate are weighed into the vial, the vial is closed with the bromobutyl rubber stopper and then purged with a flow of nitrogen. 5 ml of degassed WFI and 5 ml of a 0.9% NaCl degassed solution were then added. | | | | | |

The results reported in Table III show that the stability of the aqueous indocyanine green composition is enhanced when histidine and the compound (c) are associated to indocyanine green.

### V- Comparative compositions:

### Compositions comprising only a co-stabilizing compound (c):

**Table IV**

| | C8 | C9 | C10 | C11 | C12 |
|---|---|---|---|---|---|
| ICG (mg) | 25 | 25 | 25 | 25 | 25 |
| EDTA 4Na (mg) ^{(b)} | 1 | | | | |
| EDTA 2Na Ca (mg) | | | 2 | 2 | 2 |
| NaCl (mg) | | 45 | | 45 | 22.5 |
| Weight ratio compound (c)/ICG | 0.04 | 1.80 | 0.08 | 1.88 | 0.98 |

| Storage stability | | | | | |
|---|---|---|---|---|---|
| % area ICG prior to storage (i) | 99.70 | 99.70 | 99.70 | 99.70 | 99.70 |
| Storage (weeks) | 7 | 5 days | 5 | 5 | 5 |
| % area ICG after storage (i) | 95.60 | 89.54 | 76.58 | 94.52 | 88.88 |
| Variation (i-f) % | 4.10 | 9.53 | 23.12 | 5.18 | 10.82 |

| | | | | | |
|---|---|---|---|---|---|
| ^{(b)} Ethylenediaminetetraacetic acid tetrasodium salt hydrate. | | | | | |

The results reported in Table IV show that the compound (c) when used alone improve the storage stability of an indocyanine green aqueous composition but not as much as histidine.

### Compositions comprising components according to the prior art:

**Table V**

| | C13 | C14 | C15 | C16 | C17 | C18 | C20 | C21 | C22 |
|---|---|---|---|---|---|---|---|---|---|
| ICG (mg) | 25 | 25 | 25 | 25 | 25 | 25 | 25 | 25 | 25 |
| Methionine (mg) | 5 | | | | | | | | |
| Vitamin E (mg) (tocopherols) | | 5 | | | | | | | |
| Glutathione reduced (mg) | | | 1 | | | | | | |
| Thiolactic acid (mg) | | | | 5 | | | | | |
| Thiomalic acid (mg) | | | | | 3 | | | | |
| Thioglycolic acid (mg) | | | | | | 2 | | | |
| NaCl (mg) | | 45 | | | 45 | | | | |
| Lysine (mg) | | | | | | | 200 | | |
| Sodium bicarbonate (mg) | | | | | | | | 16 | |
| Glutaric acid (mg) | | | | | | | | | 200 |
| Weight ratio compound/ICG | 0.20 | 2 | 0.04 | 0.20 | 1.92 | 0.08 | 8 | 0.64 | 8 |

| Storage stability | | | | | | | | | |
|---|---|---|---|---|---|---|---|---|---|
| % area ICG prior to storage (i) | 99.70 | 99.07 | 99.70 | 99.07 | 99.07 | 99.07 | 99.48 | 99.48 | 99.48 |
| Storage (weeks) | 6 | 8 | 6 | 8 | 7 | 8 | 6 | 6 | 6 |
| % area ICG after storage (i) | 40.76 | 87.60 | 13.34 | 40.8 | 65.6 | 58.87 | 88.45 | 91.14 | 4.71 |
| Variation (i-f) % | 58.94 | 11.47 | 86.36 | 58.27** | 33.47 | 40.2** | 7.58 | 8.34 | 94.77 |

| | | | | | | | | | |
|---|---|---|---|---|---|---|---|---|---|
| **: A precipitation of the ICG was observed during storage. | | | | | | | | | |

The results reported in table V show that other stabilizing compounds disclosed in the prior art are insufficiently powerful with regards to the long-term stability of ICG.

### BIBLIOGRAPHY

[1] Holzer W, Mauerer M, Penzkofer A, Szeimies RM, Abels C, Landthaler M, Bäumler W. Photostability and thermal stability of indocyanine green. J Photochem Photobiol B. 1998 Dec; 47(2-3):155-64.
[2] Engel E, Schraml R, Maisch T, Kobuch K, König B, Szeimies RM, Hillenkamp J, Bäumler W, Vasold R. Light-induced decomposition of indocyanine green. Invest Ophthalmol Vis Sci. 2008 May; 49(5):1777-83.
[3] Saxena V, Sadoqi M, Shao J. Degradation kinetics of indocyanine green in aqueous solution. J Pharm Sci. 2003 Oct; 92(10):2090-7.
[4] Gathje J, Steuer RR, Nicholes KR. Stability studies on indocyanine green dye. J Appl Physiol. 1970 Aug; 29(2):181-5.
[5] Barbier f, Deweerdt ga. Chromatography and I.R. spectrography of indocyanine green. Clin Chim Acta. 1964 Dec; 10:549-54.
[6] Mindt S, Karampinis I, John M, Neumaier M, Nowak K. Stability and degradation of indocyanine green in plasma, aqueous solution and whole blood. Photochem Photobiol Sci. 2018 Sep 12; 17(9):1189-1196.
[7] Kirchherr AK, Briel A, Mäder K. Stabilization of indocyanine green by encapsulation within micellar systems. Mol Pharm. 2009 Mar-Apr;6(2):480-91.
[8] DeDora DJ, Suhrland C, Goenka S, Mullick Chowdhury S, Lalwani G, Mujica-Parodi LR, Sitharaman B. Sulfobutyl ether β-cyclodextrin (Captisol(®) ) and methyl β-cyclodextrin enhance and stabilize fluorescence of aqueous indocyanine green. J Biomed Mater Res B Appl Biomater. 2016 Oct; 104(7):1457-64.

## Claims

1. A composition comprising at least indocyanine green (a) and histidine (b) wherein the weight ratio of histidine (b)/indocyanine green (a) is greater than 0.05.

2. The indocyanine green composition as claimed in claim 1, wherein the weight ratio histidine (b) /indocyanine green (a) ranges from 0.05 to 50.

3. The indocyanine green composition as claimed in claim 2 wherein the weight ratio histidine (b)/indocyanine green (a) ranges from 0.10 to 40, preferably from 0.10 to 30, more preferably from 0.10 to 20.

4. The indocyanine green composition as claimed in any one of claims 1 to 3 further comprising at least one compound (c) chosen from ethylenediaminetetraacetic acid (EDTA) and salts thereof, cysteine, sodium chloride, dithiothreitol (DTT) and mixtures thereof.

5. The indocyanine green composition as claimed in claim 4 wherein the weight ratio of compound (c) to indocyanine green (a) ranges from 1:500 to 100:1, preferably from 1:100 to 10:1, more preferably from 1:100 to 4:1.

6. The indocyanine green composition as claimed in claim 4 or claim 5, wherein the weight ratio of compound (c) to histidine (b) ranges from 1:100 to 100:1, preferably from 1:10 to 10:1.

7. The indocyanine green composition as claimed in anyone of the preceding claims wherein it comprises up to 5% by weight of sodium iodide based on the weight of indocyanine green.

8. The indocyanine green composition as claimed in anyone of the preceding claims which is an aqueous composition.

9. The indocyanine green composition as claimed in claim 8 wherein the concentration of indocyanine green in the aqueous composition is comprised between 0.1 and 50 mg/ml.

10. The aqueous composition as claimed in claim 8 or claim 9, wherein the content of ICG in the composition prior to storage is greater than or equal to 90%, the content of indocyanine green being measured as % area by HPLC at 240 nm.

11. The indocyanine green composition as claimed in anyone of claims 1 to 7 wherein the composition is in the form of a lyophilized powder.

12. The indocyanine green composition as claimed in anyone of claims 1 to 11, for its use as a medicament or a diagnostic agent.

13. A kit comprising in a first part the composition as defined in claim 11, and in a second part an aqueous diluent.

14. A kit capable to be used for the preparation of a composition according to anyone of claim 8 to claim 10, comprising in a first part at least indocyanine green (a) and in a second part an aqueous solution comprising at least histidine, wherein the weight ratio of histidine (b)/indocyanine green (a) is greater than 0.05.

## Patentansprüche

1. Zusammensetzung, umfassend mindestens Indocyaningrün (a) und Histidin (b), wobei das Gewichtsverhältnis Histidin (b)/Indocyaningrün (a) größer ist als 0,05.

2. Indocyaningrün-Zusammensetzung nach Anspruch 1, wobei das Gewichtsverhältnis Histidin (b)/Indocyaningrün (a) in einem Bereich von 0,05 bis 50 ist.

3. Indocyaningrün-Zusammensetzung nach Anspruch 2, wobei das Gewichtsverhältnis Histidin (b)/Indocyaningrün (a) in einem Bereich von 0,10 bis 40, vorzugsweise von 0,10 bis 30, bevorzugter von 0,10 bis 20 ist.

4. Indocyaningrün-Zusammensetzung nach einem der Ansprüche 1 bis 3, ferner umfassend mindestens eine Verbindung (c), die ausgewählt ist aus Ethylendiamintetraessigsäure (EDTA) und Salzen davon, Cystein, Natriumchlorid, Dithiothreitol (DTT) und Gemischen davon.

5. Indocyaningrün-Zusammensetzung nach Anspruch 4, wobei das Gewichtsverhältnis von Verbindung (c) zu Indocyaningrün (a) in einem Bereich von 1:500 bis 100:1, vorzugsweise von 1:100 bis 10:1, bevorzugter von 1:100 bis 4:1 ist.

6. Indocyaningrün-Zusammensetzung nach Anspruch 4 oder Anspruch 5, wobei das Gewichtsverhältnis von Verbindung (c) zu Histidin (b) in einem Bereich von 1:100 bis 100:1, vorzugsweise von 1:10 bis 10:1 ist.

7. Indocyaningrün-Zusammensetzung nach einem der vorherigen Ansprüche, **dadurch gekennzeichnet, dass** sie bis zu 5 Gewichts-% Natriumiodid, bezogen auf das Gewicht von Indocyaningrün, umfasst.

8. Indocyaningrün-Zusammensetzung nach einem der vorherigen Ansprüche, die eine wässrige Zusammensetzung ist.

9. Indocyaningrün-Zusammensetzung nach Anspruch 8, wobei die Konzentration von Indocyaningrün in der wässrigen Zusammensetzung zwischen 0,1 und 50 mg/ml liegt.

10. Wässrige Zusammensetzung nach Anspruch 8 oder 9, wobei der Gehalt an ICG in der Zusammensetzung vor einer Lagerung größer als oder gleich wie 90 % ist, wobei der Gehalt an Indocyaningrün als %-Fläche durch HPLC bei 240 nm gemessen wird.

11. Indocyaningrün-Zusammensetzung nach einem der Ansprüche 1 bis 7, wobei die Zusammensetzung in Form eines lyophilisierten Pulvers ist.

12. Indocyaningrün-Zusammensetzung nach einem der Ansprüche 1 bis 11 zu ihrer Verwendung als Medikament oder Diagnostikum.

13. Kit, umfassend in einem ersten Teil die Zusammensetzung nach Anspruch 11 und in einem zweiten Teil ein wässriges Verdünnungsmittel.

14. Kit, das geeignet ist, um zur Herstellung einer Zusammensetzung nach einem der Ansprüche 8 bis 10 verwendet zu werden, umfassend in einem ersten Teil mindestens Indocyaningrün (a) und in einem zweiten Teil eine wässrige Lösung, umfassend mindestens Histidin, wobei das Gewichtsverhältnis von Histidin (b)/Indocyaningrün (a) größer ist als 0,05.

## Revendications

1. Composition comprenant au moins du vert d'indocyanine (a) et de l'histidine (b) dans laquelle le rapport pondéral d'histidine (b)/vert d'indocyanine (a) est supérieur à 0,05.

2. Composition de vert d'indocyanine selon la revendication 1, dans laquelle le rapport pondéral histidine (b)/vert d'indocyanine (a) est compris dans la plage allant de 0,05 à 50.

3. Composition de vert d'indocyanine selon la revendication 2 dans laquelle le rapport pondéral histidine (b)/vert d'indocyanine (a) est compris dans la plage allant de 0,10 à 40, de préférence de 0,10 à 30, plus préférablement de 0,10 à 20.

4. Composition de vert d'indocyanine selon l'une quelconque des revendications 1 à 3 comprenant en outre au moins un composé (c) choisi parmi l'acide éthylènediaminetétraacétique (EDTA) et des sels de celui-ci, la cystéine, le chlorure de sodium, le dithiothréitol (DTT) et des mélanges de ceux-ci.

5. Composition de vert d'indocyanine selon la revendication 4 dans laquelle le rapport pondéral du composé (c) au vert d'indocyanine (a) est compris dans la plage allant de 1:500 à 100:1, de préférence de 1:100 à 10:1, plus préférablement de 1:100 à 4:1.

6. Composition de vert d'indocyanine selon la revendication 4 ou la revendication 5, dans laquelle le rapport pondéral du composé (c) à l'histidine (b) est compris dans la plage allant de 1:100 à 100:1, de préférence de 1:10 à 10:1.

7. Composition de vert d'indocyanine selon l'une quelconque des revendications précédentes dans laquelle elle comprend jusqu'à 5 % en poids d'iodure de sodium en se basant sur le poids du vert d'indocyanine.

8. Composition de vert d'indocyanine selon l'une quelconque des revendications précédentes qui est une composition aqueuse.

9. Composition de vert d'indocyanine selon la revendication 8 dans laquelle la concentration de vert d'indocyanine dans la composition aqueuse est comprise entre 0,1 et 50 mg/ml.

10. Composition aqueuse selon la revendication 8 ou la revendication 9, dans laquelle la teneur en ICG dans la composition avant stockage est supérieure ou égale à 90 %, la teneur en vert d'indocyanine étant mesurée en % de surface par HPLC à 240 nm.

11. Composition de vert d'indocyanine selon l'une quelconque des revendications 1 à 7 dans laquelle la composition se présente sous forme d'une poudre lyophilisée.

12. Composition de vert d'indocyanine selon l'une quelconque des revendications 1 à 11, destinée à être utilisée comme médicament ou agent de diagnostic.

13. Kit comprenant dans une première partie la composition telle que définie dans la revendication 11, et dans une seconde partie un diluant aqueux.

14. Kit susceptible d'être utilisé pour la préparation d'une composition selon l'une quelconque de la revendication 8 à la revendication 10, comprenant dans une première partie au moins du vert d'indocyanine (a) et dans une seconde partie une solution aqueuse comprenant au moins de l'histidine, dans lequel le rapport pondéral d'histidine (b)/vert d'indocyanine (a) est supérieur à 0,05.
